# EUROPEAN PATENT APPLICATION

(11) **EP 4 501 210 A1**
(43) Date of publication of application: **05.02.2025**
(21) Application number: 22935713.2
(22) Date of filing: 14.12.2022
(51) Int. Cl.: A61B 3/04

(54) **DESIGN METHOD FOR SPECTACLE LENSES, PRODUCTION METHOD FOR SPECTACLE LENSES, AND MEASUREMENT METHOD FOR VISION-RELATED NUMERICAL VALUES**

(30) Priority: 28.03.2022 JP 2022051837
(71) Applicant: Hoya Lens Thailand Ltd., Pathumthani 12130 (TH)
(72) Inventor: YAMAGUCHI, Eiichiro, Tokyo 160-8347 (JP); SONEHARA, Toshiaki, Tokyo 160-8347 (JP); ITO, Ayumu, Tokyo 160-8347 (JP)
(74) Representative: Betten & Resch
(86) International application number: PCT/JP2022/046088
(87) International publication number: WO 2023/188585

(57) **Abstract**

A method for designing an eyeglass lens includes a step of setting a plurality of states in which a subject wearing a lens frame for eye examination looks downward, having the subject compare how a visual target appears, and obtaining a subjective response of the subject; and a step of designing an eyeglass lens suitable for the subject based on the subjective response.

## Description

### TECHNICAL FIELD

The present invention relates to a method for designing an eyeglass lens, a method for producing an eyeglass lens, and a method for measuring a numerical value related to vision.

### DESCRIPTION OF RELATED ART

In order to produce a prescription required for producing eyeglasses, a method is usually used in which the refractive power of a subject is subjectively tested while the subject is wearing a lens frame for eye examination. The lens frame for eye examination has a lens holding frame, and a plurality of lenses for eye examination with different properties are successively mounted on the lens holding frame to determine an appropriate eyeglass prescription for the subject.

Patent Document 1 discloses, as an example of such a lens frame for eye examination, a lens frame including a pair of lens holding devices and a temple attached to each lens holding device, a rotation means for adjusting the inclination of a temple end being provided between the lens holding devices and the temple ends.

### PRIOR ART DOCUMENT

### PATENT DOCUMENT

Patent Document 1: Japanese Patent Laid-Open Publication No. 2017-104544

### SUMMARY

### PROBLEM TO BE SOLVED BY DISCLOSURE

An object of one embodiment of the present invention is to provide a technique by which subjective responses of a subject in a plurality of states in which the subject looks downward can be reflected in the design of eyeglass lenses.

### MEANS FOR SOLVING PROBLEM

A first aspect of the present invention is a method for designing an eyeglass lens, the method including:
a step of setting a plurality of states in which a subject wearing a lens frame for eye examination looks downward, having the subject compare how a visual target appears, and obtaining a subjective response of the subject; and
a step of designing an eyeglass lens suitable for the subject based on the subjective response.

A second aspect of the present invention is the method for designing an eyeglass lens according to the first aspect,
wherein in the step of obtaining the subj ective response, the plurality of states are set using a plurality of lenses for eye examination selected to simulate an aspheric surface design.

A third aspect of the present invention is the method for designing an eyeglass lens according to the first aspect,
wherein in the step of obtaining the subj ective response, the plurality of states are set using a plurality of lenses for eye examination having different spherical powers with respect to a known addition power, and
in the step of designing an eyeglass lens, a power of a near lateral portion is corrected.

A fourth aspect of the present invention is the method for designing an eyeglass lens according to the first aspect,
wherein in the step of obtaining the subj ective response, the plurality of states are set using a plurality of lenses for eye examination having different prism amounts.

A fifth aspect of the present invention is the method for designing an eyeglass lens according to the first aspect,
wherein in the step of obtaining the subjective response, the plurality of states in which eye rotation angles are different from each other are set, and
in the step of designing an eyeglass lens, an addition power curve is corrected.

A sixth aspect of the present invention is the method for designing an eyeglass lens according to any one of the first to fifth aspects,
wherein in the step of obtaining the subjective response, a lens frame for eye examination configured such that a line of sight when the subject looks downward coincides with an optical axis of a lens for eye examination is used.

A seventh aspect of the present invention is the method for designing an eyeglass lens according to the sixth aspect,
wherein the lens frame for eye examination is configured such that when an eye rotation angle is changed, a distance from a reference point on a back side of the lens for eye examination to a corneal vertex of the subject is constant.

An eighth aspect of the present invention is a method for producing an eyeglass lens, the method including:
a step of having a subject compare how a visual target appears in a plurality of states in which the subject wearing a lens frame for eye examination looks downward, and obtaining a subjective response of the subject; and
a step of designing an eyeglass lens suitable for the subject based on the subjective response.

A ninth aspect of the present invention is a method for measuring a numerical value related to vision, the method including:
a step of having a subject compare how a visual target appears in a plurality of states in which the subject wearing a lens frame for eye examination looks downward, and obtaining a subjective response of the subject.

### ADVANTAGE OF DISCLOSURE

According to one embodiment of the present invention, a subjective response of a subject in a plurality of states in which the subject looks downward can be reflected in the design of an eyeglass lens.

### BRIEF DESCRIPTION OF DRAWINGS

FIG. 1 is a flowchart showing one example of a method for designing an eyeglass lens according to a first embodiment of the present invention.
FIG. 2 is a diagram showing a state in which a numerical value related to vision is measured in a step S100 of measuring a numerical value related to vision in a downward gaze, according to the first embodiment of the present invention.
FIG. 3 is a perspective view showing a lens frame for eye examination according to the first embodiment of the present invention.
FIG. 4 is a front view showing the lens frame for eye examination according to the first embodiment of the present invention.
FIG. 5 is a side view showing the lens frame for eye examination according to the first embodiment of the present invention.
FIG. 6 is a side view showing a state in which the lens frame according to the first embodiment of the present invention is worn by a subject.
FIG. 7 is a diagram showing how the lens frame according to the first embodiment of the present invention is adjusted such that a rotation center portion of an angle-adjustable connection part coincides with the center of eye rotation.
FIG. 8 is a diagram showing how a front temple part is rotated downward by the angle-adjustable connection part, according to the first embodiment of the present invention.
FIG. 9(a) is a graph showing a result of power simulation according to Example 1 of the present invention, and FIG. 9(b) is a graph showing mean power error and astigmatism according to Example 1 of the present invention.
FIG. 10(a) is a graph showing an aspheric surface design that prioritizes mean power error according to Example 1 of the present invention, and FIG. 10(b) is a graph showing an aspheric surface design that prioritizes astigmatism according to Example 1 of the present invention.
FIG. 11(a) is a diagram showing a power distribution prior to correction according to Example 3 of the present invention, and FIG. 11(b) is a diagram showing a power distribution after correction according to Example 3 of the present invention.
FIG. 12 is a graph showing an addition power curve according to Example 5 of the present invention.

### DETAILED DESCRIPTION OF THE DISCLOSURE

### <Finding by the inventors>

First, findings obtained by the inventors of the present invention will be described. When determining an eyeglass prescription for a subject, usually, lenses for eye examination are used to subjectively measure visual acuity. At this time, most measurements are performed in a state in which the subject looks straight ahead. On the other hand, examples of a lens through which the line of sight is directed in a direction different from the direction in which a subject wearing the lens looks straight ahead include progressive addition lenses with which a subject can see at a plurality of different distances along with eye rotation, from far to intermediate and near positions, and it is conceivable that, even with a single-vision lens, vision may vary through a peripheral portion and a center portion of the lens. Note that "eye examination" used in this specification is a method for measuring a numerical value related to vision, and refers to a series of operations in which a subject visually recognizes a target, and a refractive value, a prism value, and the like are determined based on the subjective response.

When humans look at an object below, whether intentionally or unintentionally, they not only turn their head but also look down to some extent with downward rotation of the eyes to see the object. However, there are very few cases where subjective responses are acquired while realizing such a condition, and reflected in the design of eyeglass lenses.

The inventors of the present invention conducted intensive research into the above-mentioned problems. As a result, it was found that by setting a plurality of states in which a subject looks downward and obtaining subjective responses of the subject, it is possible to design an eyeglass lens that is more suitable for the subject. Accordingly, it is possible to design an eyeglass lens in consideration of optically occurring (off-axis) aberrations and prism effects when measuring power required for producing an eyeglass lens. Further, it can also accommodate the possibility of physiological differences in vision between a straight-ahead gaze and a downward gaze occurring depending on the direction of the line of sight and the way of looking downward.

### [Details of Embodiments of the Present Invention]

Next, embodiments of the present invention will be described below with reference to the drawings. Note that the present invention is not limited to these examples, but rather is defined by the claims, and is intended to include all modifications within the meaning and scope equivalent to the claims.

The lens for eye examination mentioned in this specification (also referred to as a "optometric lens" hereinafter) has an object-side surface and an eye-side surface. The "object-side surface" is the surface located on the object side when a lens frame for eye examination provided with a lens for eye examination is worn by a subject, and is also referred to as a "front side". The "eye-side surface" is the surface on the opposite side, that is to say, the surface located on the eye side when the lens frame for eye examination provided with the lens for eye examination is worn by the subject, and is also referred to as a "back side". This relationship also applies to an eyeglass lens and a lens base material that forms the substrate of an eyeglass lens. In other words, the eyeglass lens and the lens base material also have an object-side surface and an eye-side surface.

In this specification, a downward gaze means, for example, a state other than a straight-ahead gaze (primary position), in which eyes are rotated downward (including downward right and downward left) and binocular single vision is achieved. In particular, in the following embodiments, a case will be described where the eye rotation angle (the angle at which an eye is rotated vertically downward from the position of the straight-ahead gaze) is 10 degrees or more and 40 degrees or less.

### <First Embodiment of the Present Invention> (1) Method for designing eyeglass lens

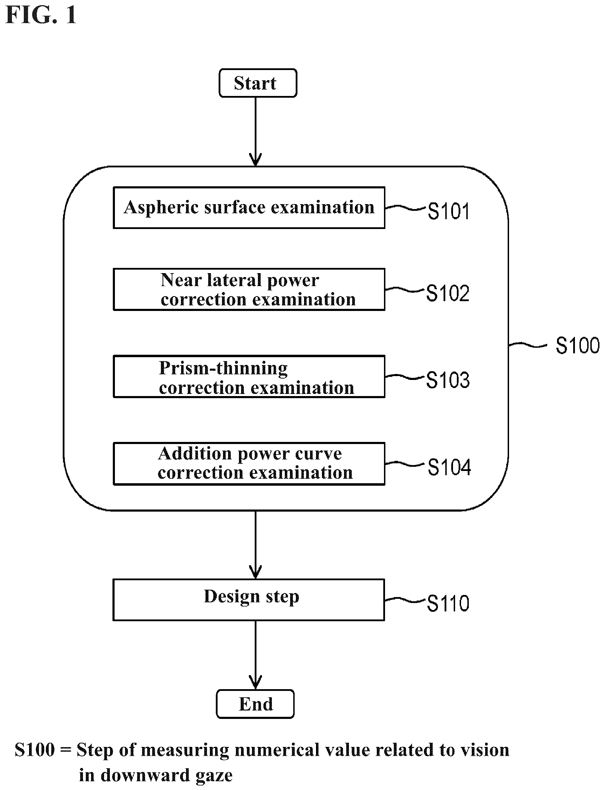

First, a method for designing an eyeglass lens according to this embodiment will be described. FIG. 1 is a flowchart showing one example of a method for designing an eyeglass lens according to this embodiment. As shown in FIG. 1, the method for designing an eyeglass lens according to this embodiment includes, for example, a step S100 of measuring a numerical value related to vision in a downward gaze and a design step S110. The step S100 of measuring a numerical value related to vision in a downward gaze includes, for example, an aspheric surface correction examination S101, a near lateral power correction examination S102, a prism-thinning correction examination S103, and an addition power curve correction examination S104.

### (Step S100 of Measuring Numerical Value related to Vision in Downward Gaze)

The step S100 of measuring a numerical value related to vision in a downward gaze is, for example, a step of setting a plurality of states in which a subject wearing a lens frame for eye examination looks downward, having the subject compare how a visual target appears, and obtaining a subjective response of the subject. As shown in FIG. 1, the step S100 of measuring a numerical value related to vision in a downward gaze includes a plurality of examinations, but all of them do not need to be performed and it is sufficient to perform at least any of them as needed.

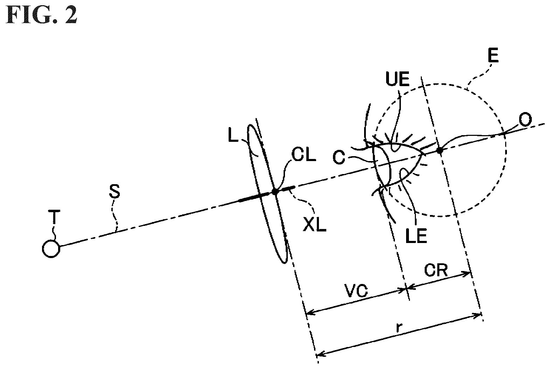

In the step S100 of measuring a numerical value related to vision in a downward gaze, it is preferable to use a lens frame for eye examination configured such that the line of sight when the subject looks downward coincides with an optical axis of the lens for eye examination. FIG. 2 is a diagram showing a state in which a numerical value related to vision is measured in the step S100 of measuring a numerical value related to vision in a downward gaze. As shown in FIG. 2, when measuring a numerical value related to vision in a downward gaze, the subject directs his/her line of sight S downward at a predetermined angle relative to a horizontal direction. At this time, an optometric lens L is rotated around the center O of eye rotation by the same angle as the rotation angle of an eye (eyeball) E while maintaining a distance r between a reference point CL on the back side of the optometric lens and the center O of eye rotation. The distance r can be calculated as a sum of a distance VC from the reference point CL on the back side of the optometric lens to a vertex of the cornea C of the eye E of the subject and a distance CR from the vertex of the cornea C to the center O of rotation of the eye E. As a result, the distance from the reference point CL on the back side of the optometric lens L to the vertex of the cornea C is equal to the distance VC in a horizontal gaze. Also, the line of sight S coincides with an optical axis XL of the optometric lens L. Accordingly, it is possible to perform highly accurate measurement without blur or prism due to aberrations.

Further, in the step S 100 of measuring a numerical value related to vision in a downward gaze, for example, it is preferable to use a lens frame for eye examination configured such that when the eye rotation angle is changed, the distance from the reference point on the back side of the lens for eye examination to the vertex of the cornea of the subject is constant. As a result, the center of rotation of an eye can coincide with the rotation center of the lens frame for eye examination, and thus it is possible to perform highly accurate measurement.

A specific configuration of the lens frame for eye examination described above will be described in detail in (2) Configuration of Lens Frame for Eye Examination. Also, a method for using a lens frame for eye examination, a method for presenting a visual target, and the like will be described in detail in (3) Method for Using Lens Frame for Eye Examination.

### (Design Step S 110)

The design step S 110 is, for example, a step of designing an eyeglass lens suitable for the subject based on the subjective response obtained in the step S 100 of measuring a numerical value related to vision in a downward gaze.

The type of eyeglass lens designed in the design step S 110 is not particularly limited, and may be a single-vision lens or a progressive addition lens.

In the design step S110, it is preferable to correct various properties of the eyeglass lens (e.g., the amount of asphericity and the amount of prism thinning) according to examinations performed in the step S100 of measuring a numerical value related to vision in a downward gaze. This makes it possible to reflect a difference in vision in a downward gaze in the design of the eyeglass lens, thus designing an eyeglass lens that is more suitable for the subject. Hereinafter, each examination performed in the step S100 of measuring a numerical value related to vision in a downward gaze and various type of correction performed in the design step S110 will be described.

### (Aspheric Surface Correction Examination S101)

In general, in a minus spherical lens, the power in the meridian direction is stronger than in the circumferential direction, and thus the power in the downward gaze state is equivalent to that looking through a lens in which the absolute value of the refractive power in both principal meridians is larger than in the 90 degree direction than in the 180 degree direction. When performing aspheric surface correction on such a lens, a method is used to minimize either the power error or astigmatism, or an index that balances the power error and astigmatism. However, the perception of blur varies from person to person and also depending on what index is used. According to the method for designing an eyeglass lens of this embodiment, the perception of blur when a subject looks downward can be reflected in an aspheric surface design of an eyeglass lens.

In the aspheric surface correction examination S101, it is preferable to set a plurality of states in which the subject looks downward, using a plurality of lenses for eye examination selected to simulate a plurality of aspheric surface designs, have the subject compare how a visual target appears, and obtain subjective responses of the subject. Specifically, for example, it is sufficient to have the subject compare how the visual target appears when astigmatism is added in the astigmatic axis 90-degree and 180-degree directions to the prescribed power corrected for the straight-ahead gaze, in the state in which the subject looks downward and obtain the subjective responses of the subject. As a result, it is possible to design the amount of asphericity suitable for the subject.

When the aspheric surface correction examination S101 is performed in the step S100 of measuring a numerical value related to vision in a downward gaze, it is preferable to correct, for example, the amount of asphericity of the eyeglass lens in the design step S110. Specifically, for example, the amount of asphericity is preferably increased (i.e., astigmatism is minimized) when the addition of astigmatism with an astigmatic axis of 90 degrees is preferred, and the amount of asphericity is preferably reduced (i.e., power error is minimized) when the addition of astigmatism with an astigmatic axis of 180 degrees is preferred. As a result, it is possible to design an eyeglass lens that provides the subject with a wearing comfort that is more favorable to the subject.

### (Near Lateral Power Correction Examination S102)

In general, addition power of a progressive addition lens is determined from the prescribed distance and near powers corrected for the straight-ahead gaze. However, with the progressive addition lens, when looking downward, the influence of eyelids and eye muscles is different from when looking straight-ahead, and thus, it is conceivable that when measuring the power particularly in a near downward gaze, the measured power is the power obtained in hyperopia or myopia compared to that in the straight-ahead gaze. According to the method for designing an eyeglass lens of this embodiment, the difference in vision when the subject looks downward at a near object can be reflected in the near lateral power of the progressive addition lens.

In the near lateral power correction examination S102, it is preferable to set a plurality of states in which the subject looks downward, using a plurality of lenses for eye examination having different spherical powers for a known addition power, have the subj ect compare how a visual target appears, and obtain subjective responses of the subject. As a result, it is possible to design a near lateral power suitable for the subject.

When the near lateral power correction examination S102 is performed in the step S100 of measuring a numerical value related to vision in a downward gaze, it is preferable to correct, for example, the near lateral power while maintaining a known addition power in the design step S110. Specifically, for example, when a spherical power larger than a known addition power is preferred, it is preferable to make a power change in the horizontal direction from a near measurement point more gradual than usual (i.e., to increase the near lateral power). On the other hand, when a spherical power smaller than a known addition power is preferred, it is preferable to make a power change in the horizontal direction from a near measurement point sharper than usual (i.e., to reduce the near lateral power). As a result, it is possible to improve a wearing comfort during a near downward gaze while maintaining a basic design of the progressive addition lens.

### (Prism-Thinning Correction Examination S103)

Prism thinning is applied to many progressive addition lenses in order to eliminate or adjust unevenness in the lens thickness in the vertical direction. In general, the maximum amount of prism thinning that results in the most uniform edge thickness is often set according to the addition power and the like, and most of the prisms are oriented in the base-down direction. Therefore, these prisms cause optical effects, such as making the ground appear to float or altering the perception of distance when looking downward. According to the method for designing an eyeglass lens of this embodiment, the difference in vision when the subject looks downward can be reflected in the amount of prism thinning of the progressive addition lens. Also, by using a lens frame for eye examination configured such that the line of sight when the subject looks downward coincides with the optical axis of the lens for eye examination, there is no need to take into consideration the prism effect of off-axis light rays, and more accurate measurement can be performed.

In the prism-thinning correction examination S103, it is preferable to set a plurality of states in which the subject looks downward, using a plurality of lenses for eye examination having different prism amounts, have the subject compare how a visual target appears, and obtain subjective responses of the subject. As a result, it is possible to design the amount of prism thinning suitable for the subject.

When the prism-thinning correction examination S103 is performed in the step S100 of measuring a numerical value related to vision in a downward gaze, it is preferable to correct, for example, the amount of prism thinning of the progressive addition lens in the design step S110. As a result, it is possible to improve a wearing comfort of the progressive addition lens.

### (Addition Power Curve Correction Examination S 104)

For example, when checking how the subject sees through a lens at the angle of downward rotation amount of an eye estimated from the progressive zone length (hereinafter, also referred to as an "estimated eye rotation angle"), if the image still appears blurred or distorted even when using a lens frame for eye examination configured to be capable of suppressing the influence of off-axis aberrations of the lens, it is highly likely that this is caused by individual physiological factors that are not related to the lens. According to the method for designing an eyeglass lens of this embodiment, the difference in vision when the subject looks downward can be reflected in the addition power curve of the progressive addition lens. Also, by using a lens frame for eye examination configured such that the line of sight when the subject looks downward coincides with the optical axis of the lens for eye examination, it is possible to eliminate the influence of off-axis aberrations of the lens for eye examination and allow the subject to see a change in vision caused by the influence of eye rotation.

In the addition power curve correction examination S104, it is preferable to set a plurality of states in which the subject looks downward such that the eye rotation angle varies, have the subject compare how a visual target appears, and obtain subjective responses of the subject. Specifically, for example, it is sufficient to have the subject compare how the visual target appears at an estimated eye rotation angle and at an eye rotation angle sharper than the estimated eye rotation angle, and to obtain subjective responses of the subject. As a result, it is possible to design an addition power curve suitable for the subject.

When the addition power curve correction examination S104 is performed in the step S100 of measuring a numerical value related to vision in a downward gaze, it is preferable to correct, for example, the addition power curve of a progressive addition lens in the design step S 110. Specifically, for example, when an eye rotation angle that is sharper than the eye rotation angle based on the addition power curve is preferred, it is preferable to correct the addition power curve (e.g., shorten the progressive zone length) such that the rate of increase (slope) of the addition power curve is increased and a desired addition power is obtained at a stage where the distance of eye movement is short. Further, when an eye rotation angle that is more obtuse than the eye rotation angle based on the addition power curve is preferred, it is preferable to correct the addition power curve (e.g., lengthening the progressive zone length) such that the rate of increase (slope) of the addition power curve is reduced and the distance of eye movement is long until a desired addition power is obtained. As a result, it is possible to improve a wearing comfort of the progressive addition lens.

Through the above steps, a subjective response of a subject in a plurality of states in which the subject looks downward can be reflected in the design of an eyeglass lens, making it possible to design an eyeglass lens suitable for the subject. Note that the present invention can also be applied as a method for measuring a numerical value related to vision. In this case, the step S100 of measuring a numerical value related to vision in a downward gaze may be performed, and the design step S110 may be omitted. Also, the present invention can also be applied as a method for producing an eyeglass lens. In this case, the eyeglass lens may be produced through the above steps. Steps other than the steps described above in this specification may be realized using a known technique.

### (2) Configuration of Lens Frame for Eye Examination

Next, a configuration of a lens frame for eye examination according to this embodiment (simply referred to as a "lens frame" hereinafter) will be described with reference to the drawings. The lens frame according to this embodiment is a lens frame that can be favorably used in the above-described step S100 of measuring a numerical value related to vision in a downward gaze.

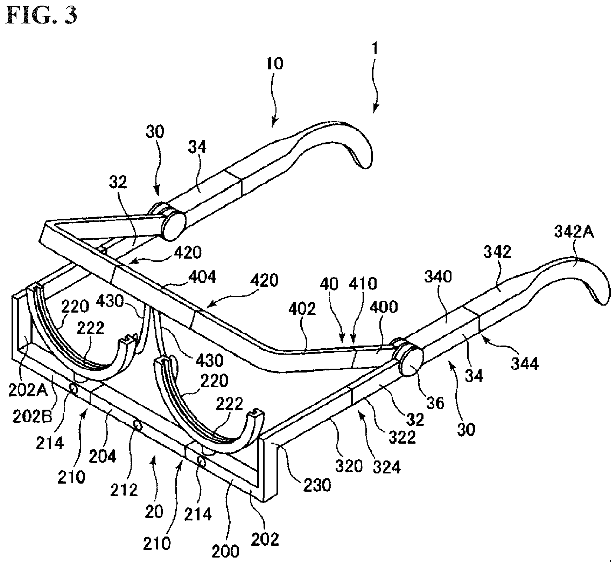

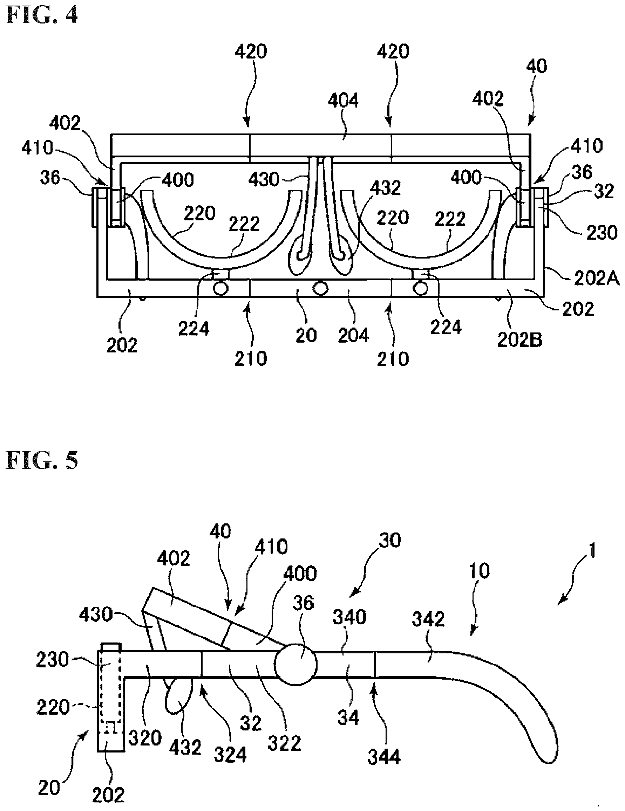

FIGS. 3 to 5 show the lens frame according to this embodiment, where FIG. 3 is a perspective view, FIG. 4 is a front view, and FIG. 5 is a side view. As shown in FIGS. 3 to 5, a lens frame 1 of this embodiment includes a frame body 10 and a lens holding member 20.

The frame body 10 includes a pair of temple members 30 and a nose rest frame 40.

Each temple member 30 includes a front temple part 32, a rear temple part 34, and an angle-adjustable connection part 36 that rotatably connects the front temple part 32 to the rear temple part 34.

A front portion of the rear temple part 34 extends linearly in the front-rear direction, and a rear end portion thereof is curved downward. The rear temple part 34 includes a first rear temple member 340 located at the front, and a second rear temple member 342 located at the rear.

The first rear temple member 340 has a hollow rectangular cross section and extends linearly. The second rear temple member 342 has a hollow rectangular cross section, and a curved portion 342Athat curves downward is formed at the rear end portion thereof. The curved portion 342A of the second rear temple member 342 functions as an earpiece when the subject wears the lens frame 1.

The width and height of the inner surface of a cross section of the second rear temple member 342 are respectively and approximately equal to the width and height of the outer surface of a cross section of the first rear temple member 340. Also, the rear end portion of the first rear temple member 340 is accommodated in a front end portion of the second rear temple member 342 in a nested manner. Such a nested structure allows the second rear temple member 342 to move in the front-rear direction relative to the first rear temple member 340. That is, this nested structure functions as a length adjustment mechanism 344 for adjusting the length of the rear temple part 34. Note that the configuration of the length adjustment mechanism of the rear temple part is not limited to a nested structure, and any appropriate configuration for adjusting and maintaining the length may be adopted, for example, such as a configuration in which a slit is formed in one of the first rear temple member 340 and the second rear temple member 342, a protrusion is formed on the second rear temple member 342, and thus the protrusion can slide within the slit. Note that cross-sectional shapes of the first rear temple member 340 and the second rear temple member 342 are not limited to a rectangle, and any appropriate shape such as a circle can be adopted.

The front temple part 32 has a shape extending linearly forward. The front temple part 32 includes a first front temple member 320 located at the front, and a second front temple member 322 located at the rear.

The first front temple member 320 has a hollow rectangular cross section and extends linearly.

The second front temple member 322 has a hollow rectangular cross section and extends linearly. The width and height of the inner surface of a cross section of the second front temple member 322 are respectively and approximately equal to the width and height of the outer surface of a cross section of the second front temple member 322. Also, the rear end portion of the first front temple member 320 is accommodated in a front end portion of the second front temple member 322 in a nested manner. Such a nested structure allows the second front temple member 322 to move in the front-rear direction relative to the first front temple member 320. That is, this nested structure functions as a length adjustment mechanism 324 for adjusting the length of the front temple part 32. Note that similarly to the configuration of the length adjustment mechanism 344 of the rear temple part 34, any appropriate configuration can be used as a configuration of the length adjustment mechanism 324 of the front temple part 32. Note that cross-sectional shapes of the first front temple member 320 and the second front temple member 322 are also not limited to a rectangle, and any appropriate shape such as a circle can be adopted.

The angle-adjustable connection part 36 rotatably connects the front temple part 32 and the nose rest frame 40 to the rear temple part 34. That is, a rotation center portion around which the front temple part 32 and the nose rest frame 40 rotate relative to the rear temple part 34 is located at the center of the angle-adjustable connection part 36. The angle-adjustable connection part 36 enables the front temple part 32 and the nose rest frame 40 to rotate relative to the rear temple part 34 within a plane defined in the up-down direction and the front-rear direction, and can maintain the angles of the front temple part 32 and the nose rest frame 40 relative to the rear temple part 34 at desired angles. A ratchet mechanism or the like can be adopted as a mechanism of such an angle-adjustable connection part 36. Note that the mechanism of the angle-adjustable connection part 36 is not limited to a configuration that allows the angles to be changed stepwise such as a latchet mechanism, and a configuration that allows the angles to be changed continuously may also be used.

The front temple part 32 can be rotated from a state in which the front temple part 32 is parallel to the rear temple part 34 in directions in which a front portion of the front temple part 32 is inclined upward and downward. A rotatable angle range of the front temple part 32 is preferably 60 degrees in the upward direction and the downward direction and more preferably 30 degrees in the upward direction and the downward direction.

The nose rest frame 40 includes a pair of first frame members 400 extending forward respectively from the angle-adjustable connection parts 36 in a horizontal view, a pair of second bent frame members 402 respectively connected to front portions of the first frame members 400, and a third lateral frame member 404 extending between the pair of second bent frame members.

A rear end of each first frame member 400 is connected to the angle-adjustable connection part 36. The first frame member 400 has a hollow rectangular cross section and extends linearly.

Each second bent frame member 402 has a hollow rectangular cross section and is bent perpendicularly between a base end portion and a leading end portion. The width and height of the inner surface of a cross section of the second bent frame member 402 are respectively and approximately equal to the width and height of the outer surface of a cross section of the first frame member 400. Also, the front end portion of the first frame member 400 is accommodated in a rear end portion of the second bent frame member 402 in a nested manner. Such a nested structure allows the second bent frame member 402 to move in the front-rear direction relative to the first front frame member 400. That is, this nested structure functions as a length adjustment mechanism 410 for adjusting and maintaining the length of the nose rest frame 40 in the front-rear direction.

The third lateral frame member 404 has a hollow rectangular cross section and extends linearly in the lateral direction. The width and height of the outer surface of a cross section of the third lateral frame member 404 are respectively and approximately equal to the width and height of the inner surface of a cross section of each second bent frame member 402. Also, both ends of the third lateral frame member 404 are accommodated in leading ends of the second bent frame members 402 in a nested manner. Such a nested structure allows the second bent frame members 402 to move in the lateral direction relative to the third lateral frame member 404, that is, the lateral width of the nose rest frame 40 can be freely changed. That is, this nested structure functions as a width adjustment mechanism 420 for adjusting the lateral width of the nose rest frame 40.

A pair of nose rest support portions 430 are attached bilaterally symmetrically to the lateral center of the nose rest frame 40. The nose rest support portions 430 are attached extending downward from the center portion of the nose rest frame 40, and nose rests 432 are attached to the leading ends thereof. When the lens frame 1 is worn, the nose rests 432 of the nose rest support portions 430 are placed in contact with both sides of the subject's nose. The nose rest support portions 430 may be made of an elastic material such as rubber, or may be made of a deformable resin.

The lens holding member 20 includes a bridge member extending between the front temple parts 32, connection portions 230 that respectively connect both ends of the bridge member 200 to front end portions of the front temple parts 32, and a pair of lens holders 220 attached to the bridge member 200.

The bridge member 200 includes a pair of first bent members 202, and a second lateral member 204 provided between the first bent members 202.

Each first bent member 202 includes a longitudinal portion 202Awhose upper end extends and is connected to a front end of the front temple part 32 via the connection portion 230, and a lateral portion 202B that is bent perpendicularly to the longitudinal portion 202A and extends inward in the lateral direction. The first bent member 202 has a hollow rectangular cross section.

The second lateral member 204 has a hollow rectangular cross section and extends linearly in the lateral direction. The width and height of the outer surface of a cross section of the second lateral member 204 are respectively and approximately equal to the width and height of the inner surface of a cross section of each first bent member 202. Also, both ends of the second lateral member 204 are accommodated in leading ends of the first bent members 202 in a nested manner. Such a nested structure allows the first bent members 202 to move in the lateral direction relative to the second lateral member 204, that is, the lateral width of the bridge member 200 can be freely changed. That is, this nested structure functions as a width adjustment mechanism 210 for adjusting and maintaining the lateral width of the bridge member 200.

Each connection portion 230 connects the front end of the front temple part 32 to the upper end of the longitudinal portion 202A of the first bent member 202 of the bridge member 200. Although the connection portion 230 can be set to any height in accordance with the length of the longitudinal portion 202A of the first bent member 202 of the bridge member 200, the connection portion 230 is located at the same height as the center of the optometric lens mounted on the lens holder 220 in this embodiment. As a result, the length of the front temple part 32 can be changed more smoothly to a predetermined length by the length adjustment mechanism 324, which will be described later.

Each lens holder 220 includes a lens holder body 222 and a columnar portion 224 that supports the lens holder body 222. The lens holder body 222 is an arc-shaped member having rigidity to hold the optometric lens, and an upper surface thereof is provided with a groove for mounting up to three optometric lenses such as trial lenses and optometric lenses.

The columnar portion 224 is round columnar, and extends downward from a lower end of the lens holder 220.

The lens holder 220 is attached to the upper surface of the lateral portion 202B of the first bent member 202 of the bridge member 200. Into the bridge member 200, a lens spacing adjustment mechanism 212 that enables the lens holder 220 to move laterally along the lateral portion 202B, and a wrap angle adjustment mechanism 214 that enables the lens holder 220 to rotate around the central axis of the columnar portion 224 are incorporated.

The lens spacing adjustment mechanism 212 and the wrap angle adjustment mechanism 214 may have a configuration in which a slit is formed in the lateral portion 202B and a base portion of the columnar portion 224 is rotatably inserted in the slit. The spacing between the lens holders 220 (the spacing between the optometric lenses) and the angles of the lens holders 220 (the angles of the optometric lenses) can be changed by rotating dial members 212 and 214.

Note that it is desirable to adopt a configuration in which the lateral movement of the lens holders 220 is performed by the lens spacing adjustment mechanism 212 such that the pair of lens holders 220 move in a bilaterally symmetric manner. A configuration or the like can be adopted as such a configuration, in which screw rods provided with threaded grooves bilaterally symmetrically are disposed within the first bent members 202 and the second lateral member, and nuts that screw onto the screw rods are attached to the lower end portions of the columnar portions 224, and thus the screw rods are rotated.

### (3) Method for Using Lens Frame for Eye Examination

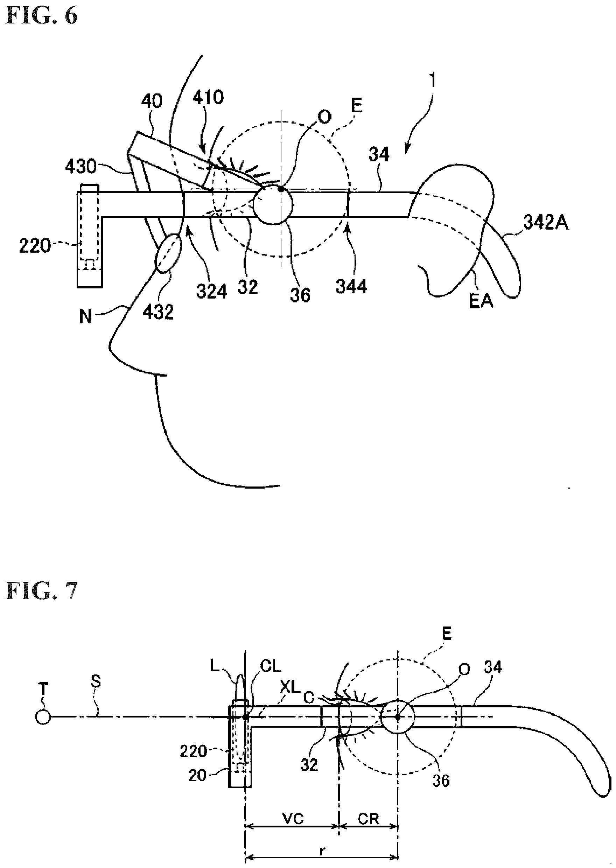

Next, a method for using a lens frame 1 will be described. First, the lens frame 1 is worn by a subject. FIG. 6 is a side view showing a state in which the lens frame according to this embodiment is worn by the subject. As shown in FIG. 6, the lens frame 1 can be worn by a subject P by bringing the nose rests 432 of the nose rest support portions 430 into contact with both sides of a nose N of the subject P, and placing the curved portions 342A of the pair of rear temple parts 34 on ears EA of the subject P. Also, at this time, the width between the pair of temple members 30 is changed in accordance with the width of the face of the subject P. The width between the pair of temple members 30 can be changed by adjusting the width of the bridge member 200 using the width adjustment mechanism 210 and the width of the nose rest frame 40 using the length adjustment mechanism 410. Also, in accordance with an interpupillary distance PD of the subject, the lens holders 220 are moved laterally by the lens spacing adjustment mechanism 212 such that reference points of the lens holders 220 coincide with the centers of the pupils of the subject.

Next, as shown in FIG. 7, the lens frame 1 is adjusted such that the rotation center portion of the angle-adjustable connection part 36 coincides with the center O of eye rotation. Specifically, the length of the rear temple part 34 is adjusted by the length adjustment mechanism 344, the length of the nose rest frame 40 is adjusted by the length adjustment mechanism 410, and the angle of the nose rest frame 40 relative to the rear temple part 34 is adjusted. In this manner, the position of the angle-adjustable connection part 36 of the lens frame can be moved in a vertical plane in the front-rear direction. Then, a rotation center axis of the angle-adjustable connection part 36 can be moved to coincide with the center O of eye rotation.

Note that this adjustment operation can be completed only with minor adjustments by adjusting the length of the rear temple parts 34, the length of the nose rest frame 40, and the angles of the nose rest frame 40 relative to the rear temple parts 34 in advance, before the lens frame 1 is worn.

The lens frame 1 can be fitted to the face of the subject P such that the rotation centers of the angle-adjustable connection parts 36 of the lens frame 1 are respectively located on the lateral sides of the centers O of eye rotation of the subject P, by fitting the lens frame 1 and performing adjustment operations as described above.

Then, an optometric lens L is attached to the lens holder 220. A maximum of three optometric lenses L are attached to the lens holder 220. Lenses for spherical power, lenses for cylindrical power, and lenses for prism are used in an appropriate combination as the optometric lenses. Also, the position of an optometric lens L is adjusted such that a horizontal line HL coincides with an optical axis XL of the optometric lens of the optometric lens L and the distance from the reference point CL on the back side of the optometric lens L to a corneal vertex is VC. As a result, the distance from the reference point CL on the back side of the optometric lens to the center O of eye rotation satisfies r=VC+CR. Note that the position of the optometric lens L can be adjusted by rotating the front temple part 32 relative to the rear temple part 34 around the angle-adjustable connection part 36 and adjusting the length of the front temple part 32 using the length adjustment mechanism 324.

Before or after the step S100 of measuring a numerical value related to vision in a downward gaze is performed, eye examination data in a horizontal gaze may be measured. Specifically, a visual target T is presented to the subject at a vision distance where the subject needs an eyeglass lens, for example, about 2 m in front of the subject, and the subject looks at the visual target T through the optometric lens in a horizontal gaze. Then, based on responses of the subject, the optometric lenses are replaced, and eye examination data in the horizontal gaze is measured while checking visual acuity or wearing comfort.

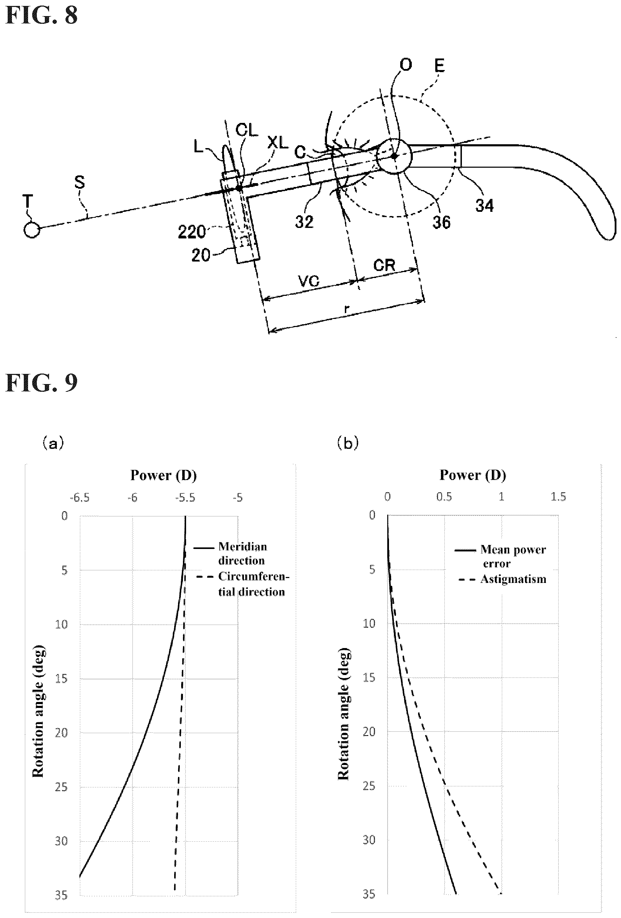

Then, as shown in FIG. 8, each front temple part 32 is rotated downward by only a specified angle, for example, 15 degrees, by the angle-adjustable connection part 36. At this time, each lens holder 220 may be rotated to a predetermined wrap angle. Then, eye examination data in a downward gaze is measured (the step S100 of measuring a numerical value related to vision in a downward gaze). Specifically, the visual target T is presented approximately 2 m in front of the subject, at a height 15 degrees below the subject. Then, the visual target T is presented to the subject through the optometric lens while keeping the subject's head still. Thereafter, based on responses of the subject, the optometric lenses are replaced, and eye examination data in the downward gaze is measured while checking visual acuity or wearing comfort.

At this time, by rotating each front temple part 32 downward by a predetermined angle around the angle-adjustable connection part 36 that coincides with the center O of eye rotation, a relative position of the optometric lens L and a pupil of the subject can be made to remain unchanged from that in a horizontal gaze. That is, the line of sight S of the subject coincides with the optical axis XL of the optometric lens L, and the distance from the reference point CL on the back side of the optometric lens L to the corneal vertex is VC.

Note that when measuring eye examination data in a downward gaze or a horizontal gaze, the subject may be instructed to look at a visual target present at a specified angle in a natural posture while allowing the subject to move his/her head, and the rotation angle of the head of the subj ect at this time may be measured by processing image data or the like, and thus the front temple parts 32 may be respectively rotated downward by the angle-adjustable connection parts 36 by an angle that is equal to the difference between the specified angle and the rotation angle of the head. Even in such a case, the line of sight S of the subject coincides with the optical axis XL of the optometric lens L, and the distance from the reference point CL on the back side of the optometric lens L to the corneal vertex is VC. Note that the rotation angle of the head of the subject can also be obtained using a front or side image of the subject or a measuring device such as a gyro sensor.

The lens frame 1 of this embodiment is capable of measuring eye examination data in both a horizontal gaze and a downward gaze. Therefore, for example, even when eye examination data in a horizontal gaze is measured before and after the step S100 of measuring a numerical value related to vision in a downward gaze, there is no need to replace the lens frame, and measurement can be smoothly performed.

### <Other Embodiments of the Present Invention>

Although embodiments of the present invention have been specifically described above, the present invention is not limited to the above-described embodiments, and various modifications can be made without departing from the gist of the present invention.

For example, the nose rest frame 40 is rotatable around the angle-adjustable connection parts 36 and its length is adjustable by the length adjustment mechanism 410 in the above embodiment. However, the nose rest frame 40 does not need to be rotatable or its length does not need to be adjustable. For example, when the nose rest support portions 430 are deformable members capable of supporting the lens frame 1, the rotation center portion of the front temple part 32 can be freely moved in the front-rear direction and the up-down direction.

### Examples

Next, examples according to the present invention will be described. These examples are merely examples of the present invention, and the present invention is not limited to these examples.

### (Example 1)

The following describes Example 1 in which the aspheric surface correction examination S101 was performed on a subject whose prescription is SPH (spherical power) of -5.50 D to design a single-vision lens with a refractive index of 1.60 and a base curve of 1. Note that, for example, a state in which the eye rotation angle in a downward gaze is 30 degrees will be simply referred to as 30-degree downward gaze to avoid complication in the following examples.

First, a power simulation was performed in the case of a spherical design, and a power distribution in the meridian and circumferential directions was calculated. The results of the simulation are shown in FIG. 9(a). A mean power error and astigmatism were calculated from the mean power and difference between powers shown in FIG. 9(a). The results are shown in FIG. 9(b). As shown in FIG. 9(b), for example, it was found that, in a 30-degree downward gaze, the mean power error was 0.46 D and astigmatism was 0.75 D. Aspheric surface design was performed in order to reduce this error, but theoretically, the astigmatism and the mean power error cannot be 0 at the same time. In view of this, in order to optimize aspheric surface design, the aspheric surface correction examination S101 was performed, and the subject compared, at a 30-degree downward gaze position, the wearing comfort of (A): lenses for eye examination combined to have SPH of - 5.25 D, CYL (cylinder power) of -0.25 D, and AX (astigmatic axis) of 180 degrees, with the wearing comfort of (B): a lens for eye examination with SPH of -5.25 D.

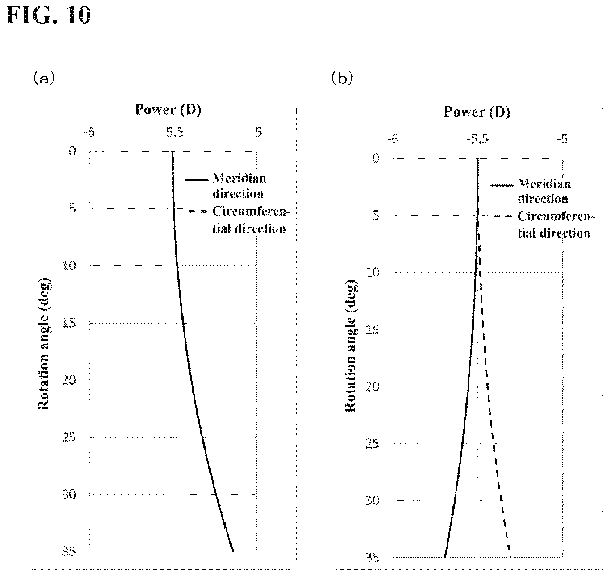

When the subject responded that (A) is better, the aspheric surface design that prioritizes mean power error, as shown in FIG. 10(a), can be applied, and when the subject responded that (B) is better, the aspheric surface design that prioritizes astigmatism, as shown in FIG. 10(b), can be applied. Based on the above, it was confirmed that it is possible to design lenses that are optimized for the eye's condition in a downward gaze.

Note that, in this example, optimization of the aspheric surface design for distance vision in both cases has been described as examples. However, the lens power for comparing wearing comfort may be determined based on a simulation of the aspheric surface design performed in a state in which a target distance is varied, for example. Also, in a case where the response is that a state closer to spheric surface design is preferred, there is no need to perform an aspheric surface design. For example, if the best wearing comfort is obtained when a subject wore (C): lenses for eye examination combined to have SPH of -5.50 D, CYL of -0.75 D, and AX of 180 degrees, then it may be determined that a design without adding an aspheric surface is optimal, and a lens may be designed accordingly.

### (Example 2)

Similarly to Example 1, the following describes Example 2 in which the aspheric surface correction examination S101 was performed on a subject whose prescription is SPH of -5.50 D to design a single-vision lens with a refractive index of 1.60 and a base curve of 1, using a method focused on a change in the amount of astigmatism as a simpler method.

If a negative cylinder power is added to (A) in the AX direction of 90 degrees, an astigmatism component will be offset, resulting in a wearing comfort closer to (B). If a negative cylinder power is added thereto in the AX direction of 180 degrees, then, the amount of astigmatism will be increased, resulting in a wearing comfort close to (C). Although the direction of this change in wearing comfort is maintained even when (A) is a prescription value, it is more preferable to prevent the spherical equivalent power from changing by using an added lens. In view of this, the aspheric surface correction examination S101 is performed, and a subject compared three states: the subject wore a lens for eye examination with SPH of -5.50 D at a 30-degree downward gaze position, and a lens with SPH of +0.25 D and CYL of -0.50 D was added in the AX direction of 90 degrees and in the AX direction of 180 degrees.

In this case, an aspheric surface design and a spheric surface design that prioritize astigmatism can be respectively assigned to a subject who said that the AX direction of 90 degrees was the best, and a subject who said that the AX direction of 180 degrees was best, and an aspheric surface design prioritizing power can be assigned to a subject who said that no addition of lens was best, to achieve a prescribed power. Based on the above, similarly to Example 1, it was confirmed that it is possible to design lenses that are optimized for the eye's condition in a downward gaze. Because there is a continuous change between a spherical design and an aspheric surface design prioritizing power, and between an aspheric surface design prioritizing power and an aspheric surface design prioritizing astigmatism, it is possible to realize a more favorably balanced design by changing weighting over multiple trials.

### (Example 3)

The following describes Example 3 in which the near lateral power correction examination S102 was performed on a subject whose prescription is SPH of 0.00 D and Add (addition power) of 2.00 D to design a progressive addition lens.

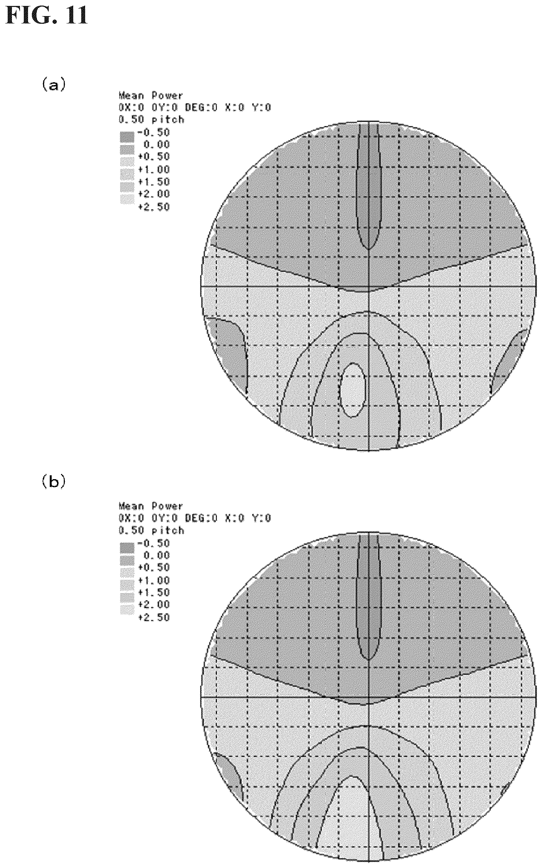

The subject wore lenses for eye examination with SPH of +2.00 D and SPH of +2.25 D while looking downward at a visual target at a near distance (about 40 cm), and compared how the visual target appeared. The vision through the lens with SPH of +2.25 D was preferred. In this case, it is conceivable that an example of a lens with Add of 2.00 D normally designed based on the prescription would be a lens with a power distribution as shown in FIG. 11(a). However, since the subject preferred a larger spherical power during downward eye rotation, a design can be adopted in which a power change in a horizontal direction from near measurement point is more gradual than usual, as shown in FIG. 11(b). From the above, it is possible to design a progressive addition lens in consideration of effects of eye rotation. In the design adopted in this example, the power of the near lateral portion used for a near downward gaze can be set stronger than in a normal design while maintaining the addition power. Thus, when the subject wears a lens, the resulting vision approaches the vision selected through the comparison using the lens frame for eye examination, thus improving wearing comfort in a near downward gaze.

Although this example was described using a case in which the near lateral power correction examination S102 is performed with one type of eye rotation angle in a downward gaze, the eye rotation angle in a downward gaze may be changed, and a subjective response of a subject for every eye rotation angle may be obtained. In this case, for example, it is possible to correct the power of the near lateral portion to be stronger or weaker than that in a normal design for every eye rotation angle while maintaining an addition power.

### (Example 4)

The following describes Example 4 in which the prism-thinning correction examination S103 was performed and a progressive addition lens with corrected amount of prism thinning was designed.

In the case of left and right progressive addition lenses with prescriptions with SPH of - 2.50 D, Add of 2.50 D, and a progressive zone length of 11 mm, a normal amount of prism thinning is 1.00 prism DN. In view of this, the prism-thinning correction examination S103 was performed on a subject with the above prescription. In a 22-degree downward gaze, the subject wore a lens for eye examination with prism DN of 1.00 and a lens for eye examination with prism DN of 0.50, and compared the wearing comfort, and the subject responded that the lens with prism DN of 0.50 was more comfortable. Also, when the lens with prism DN of 0.50 was compared with a lens with no prism, the subject responded that there was no difference in wearing comfort. In this case, a design for 0.50 prism DN can be applied. From the above, it was confirmed that it is possible to design a lens that realizes the best balance between thinness and visibility.

### (Example 5)

The following describes Example 5 in which the addition power curve correction examination S104 was performed on a subject whose prescription is SPH of 0.00 D and Add of 2.00 D, and an addition power curve for a progressive addition lens is corrected.

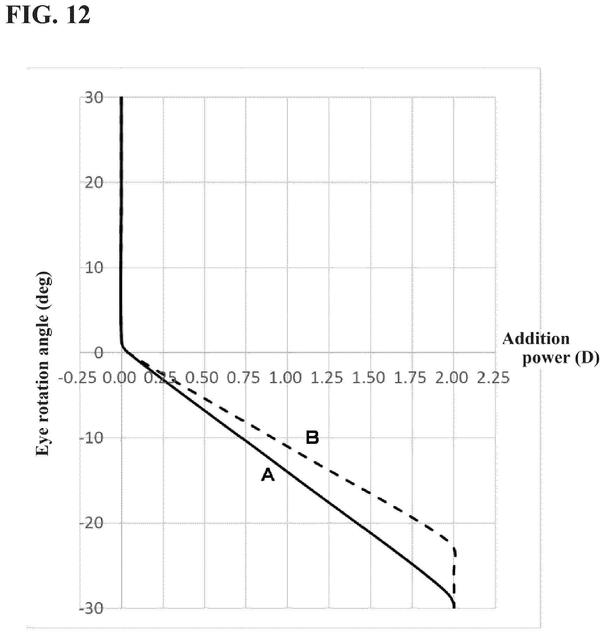

First, the subject checked the wearing comfort with a lens power of +2.00 D at a 28-degree downward gaze position. Then, the subject compared the wearing comfort in two states: a 22-degree downward gaze and a 28-degree downward gaze. When a response is received that a 22-degree downward gaze is preferred, designing an addition power curve A shown in FIG. 12 (i.e., correcting the addition power curve to shorten the progressive zone length) would be sufficient, whereas when a response is received that a 28-degree downward gaze is preferred, designing an addition power curve B shown in FIG. 12 (i.e., correcting the addition power curve to extend the progressive zone length) would be sufficient. As described above, it was confirmed that it is possible to design an addition power curve according to an eye rotation angle preferred by a subject.

### DESCRIPTION OF SIGNS AND NUMERALS

- 1: Lens frame
- 10: Lens frame body
- 20: Lens holding member
- 30: Temple member
- 32: Front temple member
- 34: Rear temple member
- 36: Angle-adjustable connection part
- 40: Nose rest frame
- 200: Bridge member
- 202: First bent member
- 202A: Longitudinal portion
- 202B: Lateral portion
- 204: Second lateral member
- 210: Width adjustment mechanism
- 212: Lens spacing adjustment mechanism (dial member)
- 214: Wrap angle adjustment mechanism (dial member)
- 220: Lens holder
- 222: Lens holder body
- 224: Columnar portion
- 230: Connection portion
- 320: First front temple member
- 322: Second front temple member
- 324: Length adjustment mechanism
- 340: First rear temple member
- 342: Second rear temple member
- 342A: Curved portion
- 344: Length adjustment mechanism
- 400: First frame member
- 402: Second bent frame member
- 404: Third lateral frame member
- 410: Length adjustment mechanism
- 420: Width adjustment mechanism
- 430: Nose rest support portion
- 432: Nose rest
- 500: Progressive addition lens
- 510: Distance portion
- 520: Near portion
- 530: Intermediate portion
- S100: Step of measuring numerical value related to vision in downward gaze
- S101: Aspheric surface correction examination
- S102: Near lateral power correction examination
- S103: Prism-thinning correction examination
- 5104: Addition power curve correction examination
- S 110: Design step

## Claims

1. A method for designing an eyeglass lens, comprising:
a step of setting a plurality of states in which a subject wearing a lens frame for eye examination looks downward, having the subject compare how a visual target appears, and obtaining a subjective response of the subject; and
a step of designing an eyeglass lens suitable for the subject based on the subjective response.

2. The method for designing an eyeglass lens according to claim 1,
wherein in the step of obtaining the subj ective response, the plurality of states are set using a plurality of lenses for eye examination selected to simulate an aspheric surface design.

3. The method for designing an eyeglass lens according to claim 1,
wherein in the step of obtaining the subj ective response, the plurality of states are set using a plurality of lenses for eye examination having different spherical powers with respect to a known addition power, and
in the step of designing an eyeglass lens, a power of a near lateral portion is corrected.

4. The method for designing an eyeglass lens according to claim 1,
wherein in the step of obtaining the subj ective response, the plurality of states are set using a plurality of lenses for eye examination having different prism amounts.

5. The method for designing an eyeglass lens according to claim 1,
wherein in the step of obtaining the subjective response, the plurality of states in which eye rotation angles are different from each other are set, and
in the step of designing an eyeglass lens, an addition power curve is corrected.

6. The method for designing an eyeglass lens according to any one of claims 1 to 5,
wherein in the step of obtaining the subjective response, a lens frame for eye examination configured such that a line of sight when the subject looks downward coincides with an optical axis of a lens for eye examination is used.

7. The method for designing an eyeglass lens according to claim 6,
wherein the lens frame for eye examination is configured such that when an eye rotation angle is changed, a distance from a reference point on a back side of the lens for eye examination to a corneal vertex of the subject is constant.

8. A method for producing an eyeglass lens, comprising:
a step of having a subject compare how a visual target appears in a plurality of states in which the subject wearing a lens frame for eye examination looks downward, and obtaining a subjective response of the subject; and
a step of designing an eyeglass lens suitable for the subject based on the subjective response.

9. A method for measuring a numerical value related to vision, comprising:
a step of having a subject compare how a visual target appears in a plurality of states in which the subject wearing a lens frame for eye examination looks downward, and obtaining a subjective response of the subject.
